# EUROPÄISCHE PATENTANMELDUNG

(11) **EP 1 338 285 A1**
(43) Veröffentlichungstag der Anmeldung: **27.08.2003**
(21) Anmeldenummer: 03000911.2
(22) Anmeldetag: 16.01.2003
(51) Int. Cl.: A61K 35/16

(54) **Plasmagel**

(30) Priorität: 04.02.2002 DE 10204405
(71) Anmelder: Surface Care GmbH, 75031 Eppingen (DE)
(72) Erfinder: Schneider, Henning, 75031 Eppingen (DE); Lang, Ekkehard, 35796 Weinbach (DE)
(74) Vertreter: Wagner, Jutta, Dr.

(57) **Zusammenfassung**

Die vorliegende Erfindung betrifft ein Plasmagel, das aus 10 Teilen Blutplasma, 0,5 bis 20 Teilen eines physiologisch verträglichen Mediums, 0,5 bis 5 Teilen Calciumchloridlösung und 1 bis 10 Teilen Glycerin oder Glycerinhydrat besteht, mit der Maßgabe, dass die Konzentration an Calciumionen im Bereich von 20 bis 60 mmol/l liegt und der Anteil Glycerin im Bereich von 1 bis 10 % beträgt. Weiterhin betrifft die Erfindung Zelltransplantate zum Wiederaufbau von geschädigtem oder zerstörtem Gewebe aus Zellkulturen, wobei die vorzugsweise in vitro kultivierten Zellen in dem Plasmagel appliziert werden. Das Plasmagel eignet sich auch für die Kultur von Zellen.

## Beschreibung

Gegenstand der vorliegenden Erfindung ist ein neuartiges Plasmagel, das aus Blut oder Blutprodukten hergestellt wird, zur Regenerierung verletzter Hautpartien, Verfahren zu seiner Herstellung sowie Zellkulturen, die aus humanem Biopsiematerial isoliert werden und in dem Plasmagel als kurzzeitig immobilisierendem Träger vorliegen.

Verletzungen der Oberhaut (Epidermis) und der darunterliegenden Lederhaut (Dermis), also Verletzungen die die Haut in ihrer gesamten Dicke schädigen, treten überwiegend bei Verbrennungen, Verätzungen, bei dermatologischen und chronischen Krankheitsbildern auf. Der permanent verbleibende Verschluss großflächiger tiefer Wunden ist nach wie vor das zentrale Problem der akuten und chronischen Wundbehandlung. Die möglichst frühzeitige Behandlung großflächiger Wunden zielt auf die Vermeidung von Flüssigkeitsverlusten und Infektionen, die Hauptursachen für die ansonsten hohe Mortalität. Die Behandlung chronischer Wunden erfordert zusätzliche Maßnahmen, die auf den Erhalt der Stabilität der epidermalen Gewebsschicht zielen und der grunderkrankungsbedingten Versorgungsstörung der Oberhaut entgegenwirken.

Gerade bei großflächigen Wunden ist ein Ersatz der untergegangenen Zellen unumgänglich. Gebräuchlich sind dabei Hauttransplantate, die entweder dem Patienten an einer unverletzten Stelle entnommen werden oder mittels Tissue engineering in vitro hergestellt werden. Obwohl diese Transplantate schon gute Ergebnisse erzielen sind mit dieser Technik noch verschiedene Nachteile verbunden. Bei Fremdgewebe bereiten Abstoßungsreaktionen Probleme, autologe Transplantate sind häufig nur begrenzt oder bei Schwerstverletzten gar nicht verfügbar. Zudem lassen die Ergebnisse in kosmetischer Hinsicht häufig zu wünschen übrig.

Lösungen zum Verschluss oder zum temporären Ersatz zerstörten Gewebes und zur allgemeinen Stimulierung der Wundheilung und wurden daher entwickelt, um solche problematischen, flächigen Wunden durch physiologische Methoden abdecken, deren Heilung verbessern und verlorengegangenes Gewebe ersetzen zu können.

Die WO 00/32207 beschreibt ein Verfahren zur Fixierung von Zellen an einer Zieloberfläche, bei dem suspendierte Zellen aus einer Zellkultur und eine Zweikomponenten Mischung aus einem nicht-polymeren Fibrin und einer Substanz zu dessen Umsetzung zu polymerem Fibrin auf die Zieloberfläche aufgesprüht werden, wobei die beiden Komponenten nach dem Aufsprühen einen Fibrinkleber bilden, der die Zellen auf dem Zielgewebe fixiert. In Beispiel 3 wird die Behandlung von Wunden mit Keratinozyten als Zellen beschrieben.

Die Herstellung von Fibrinkleber ist in der EP 952 242 offenbart. Zur Herstellung des zunächst nicht-polymeren Fibrins ist ein mehrstufiges Verfahren erforderlich, bei dem man zunächst Fibrinogen aus Blut oder Zellkulturen gewinnt, dieses dann durch ein thrombinartig wirkendes Enzym zu einem löslichen polymeren Fibrin umsetzt und anschließend von dem Enzym trennt und solubilisiert. Als zweite Komponente, die die Umsetzung zu unlöslichem Fibrinpolymer bewirkt, werden vorzugsweise Calciumionen eingesetzt.

Das in der WO 00/32207 beschriebene Verfahren erfordert, dass zur Applikation drei verschiedene Komponenten, nämlich Zellkultur, nicht-polymeres Fibrin und Thrombin in Calciumionenlösung auf Wunden aufgebracht werden. Nachteilig kann sein, das die im verwendeten Fibrin eingeschlossenen Zellen vom Organismus unter Zuhilfenahme von zellulären enzymatischen Prozessen befreit werden müssen. Dies gilt insbesondere dann, wenn der Fibrinabbau durch den Zusatz von Stabilisatoren (z.B. Aprotinin) weiter verzögert wird. Potentiell kann kreuzvernetztes Fibrin, besonders bei einer Verwendung in dickeren Schichten, eine Gewebsreorganisation als Diffusions- oder Zellmigrationsbarriere behindern.

Weitere Verfahren zur Herstellung von Fibrinkleber sind in der WO 86/01814 und der US 5,962,420 beschrieben. Ein in der ES 2 132 027 A1 beschriebenes Verfahren betrifft eine Fibrinpräparation unter Hinzunahme von Zellen (Fibroblasten) aus der Zellkultur, die mit polymerem Fibrin auf Wunden aufgebracht werden. Die Herstellung der Fibrinpräparation erfolgt durch Kryopräzipitation von Blut oder Plasma, was ein sehr aufwändiges Verfahren ist.

Es bestand daher die Aufgabe, die Möglichkeiten der Wundbehandlung weiter zu verbessern bzw. alternative Behandlungsmöglichkeiten bereitzustellen.

Überraschend wurde nun gefunden, dass Zellkulturen, die in einem aus Blut oder Blutplasma hergestellten Plasmagel, welches Fibrinogen in physiologischer Konzentration enthält, auf Wunden aufgebracht werden, zu einer beschleunigten Wundheilung führen. Von besonderer Wichtigkeit ist die Erkenntnis, dass diese Art der Applikation von Zellsuspensionen keine konzentrierte langzeitstabile Trägermatrix erfordert.

Die vorliegende Erfindung stellt demgemäss zur Lösung der gestellten Aufgabe ein Plasmagel zur Wundbehandlung gemäß Anspruch 1 und ein Verfahren zu seiner Herstellung gemäß Anspruch 5 bereit.

Das erfindungsgemäße Plasmagel wird aus allogenem, vorzugsweise autologem Blut oder Blutplasma gewonnen. Bei einer Gewinnung aus Blut wird in einem ersten Schritt das Blut unter Zusatz eines gerinnungshemmenden Mittels, z.B. durch Plasmapherese oder Vollblutabnahme, entnommen. Das gerinnungshemmende Mittel ist erforderlich, damit das Blut während der Entnahme nicht gerinnt und die Gerinnungsfaktoren funktionsfähig bleiben. Als gerinnungshemmendes Mittel haben sich beispielsweise Heparin, Dextran und insbesondere Zitrat bewährt. Als Blutplasma kann z.B. sogenanntes fresh frozen Plasma (FFP) oder ein anderes humanes Vollblutderivat eingesetzt werden. Die zellulären Anteile des Blutes können gewünschtenfalls in an sich bekannter Weise, beispielsweise durch zentrifugieren, abgetrennt werden. Im folgenden werden sowohl das von den zellulären Anteilen befreite als auch das mit den zellulären Bestandteilen verwendete Produkt als Plasma bezeichnet.

Das so gewonnene Plasma wird mit einem physiologisch verträglichen Medium im Verhältnis 0,1 bis 2 : 1 verdünnt und mit Calciumchloridlösung und Glycerin versetzt. Als physiologisch verträgliches Medium eignen sich Dulbecco modified Eagle medium (DMEM), Leibovitz L-15 (von Gibco) und insbesondere physiologische Kochsalzlösung oder Ringerlactat. Es wird soviel Calciumchloridlösung zugegeben, dass das Plasmagel 30 bis 50 mmol, vorzugsweise 40mmol Calciumionen pro I enthält.

Das Glycerin kann auch als Glycerinhydrat zugefügt werden, die Konzentration sollte etwa 1 bis 10 Gew-%, bevorzugt 1 bis 6 Gew-% und insbesondere 1 bis 3 Gew-% betragen. Das Glycerin wirkt als physiologischer Emulgator, wobei die erfindungsgemäß angewandte Konzentration die Viskosität der Lösung erhöht und die Gerinnungskaskade, die durch Thrombinzusatz in vitro sonst ausgelöst wird, verlangsamt. Somit ist ein Klebeeffekt ermöglicht und alle im Blut enthaltenen physiologisch bedeutsamen Bestandteile stehen zur Wundheilungsförderung zur Verfügung. Der Klebeeffekt des erfindungsgemäßen Plasmagels beruht auf dem im Humanplasma in einer Konzentration von typischerweise 200 bis 300 mg/ml enthaltenen Fibrinogen.

Das erfindungsgemäße Plasmagel eignet sich einerseits als heilungsfördernde Wundabdeckung zur Erstversorgung. Insbesondere können aber in dem erfindungsgemäßen Plasmagel verschiedenste Zellen bzw. Zellkulturen auf geschädigtes Gewebe aufgebracht werden. Weiterhin eignet sich das erfindungsgemäße Plasmagel auch als Substratunterlage für die Zellkultur.

Das Plasmagel wird erfindungsgemäß aus allogenem oder autologem Blut oder Blutplasma hergestellt, indem dieses in einen sterilen Rührbehälter überführt wird und zu je 10 ml Plasma etwa 1 bis 20 ml, vorzugsweise 4 bis 10 ml eines physiologisch verträglichen Mediums, gegeben werden. Dann werden unter Rühren 0,5 bis 5 ml, vorzugsweise 1 bis 3 ml einer Calciumchloridlösung zugesetzt, wobei in dem fertigen Plasmagel eine Konzentration an Calciumionen von 30 bis 50 mmol/l, insbesondere 40 mmol/l, eingestellt wird. Dem Ansatz wird anschließend Glycerin oder Glycerinhydrat bis zu einer Endkonzentration von 1 bis 10 Gew-%, vorzugsweise 1 bis 6 Gew-% zugefügt. Das Gemisch wird solange gerührt, bis es zu einer mäßig zähflüssigen Konsistenz geliert. Das resultierende Plasmagel ist sowohl im Kühlschrank als auch unter Brutraumbedingungen lagerstabil, es kann vorzugsweise im Kühlschrank aufbewahrt werden.

Sofern eine festere Struktur, z.B. ein Gelfilm, erwünscht ist, können dem Plasmagel noch 5 bis 5000 Einheiten Thrombin, vorzugsweise 50 - 500 I.E. Thrombin zugefügt werden. Alternativ kann das Thrombin auch bei der Applikation des erfindungsgemäßen Plasmagels, z.B. durch Verwendung eines Mehrkomponentenzerstäubers, zugesetzt werden.

Es ist weiterhin bevorzugt, dem Plasmagel xenogenes, allogenes oder auch autologes Kollagen in nativer oder säuregelöster Form, z.B. aus azellulärer Dermis (Alloderm ®, Epiflex ®) gewonnen, in einer Menge von 1 bis 80 Gew-%, vorzugsweise 10 - 30 Gew-%, zuzusetzen.

Bei einem Einsatz des erfindungsgemäßen Plasmagels als Wundabdeckung oder als kurzzeitig immobilisierender Träger für Zellen oder Zellkulturen wird nach der Applikation auf die zu behandelnde Wunde durch die im Gewebe vorhandenen natürlichen Gerinnungsfaktoren eine weitere Verfestigung des Plasmagels unter Ausbildung einer festen Verbindung mit der Wundoberfläche ausgelöst. Auf die Verwendung von zusätzlichen Substanzen, z.B. Aprotinin zur Stabilisierung des Fibrinklebers, wird ausdrücklich verzichtet.

Der Gelfilm kann je nach Fragestellung mit pharmazeutisch wirksamen Substanzen, Arzneimittelrelease-Systemen oder bekannten, die Wundheilung stimulierenden Faktoren oder Kombinationen daraus verbunden werden.

In einer weiteren Ausführungsform können dem Plasmagel Thrombozyten, insbesondere autologe Thrombozyten, als wundheilungsfördernde Bestandteile, vorzugsweise in einer Konzentration von 10.000 bis 1.000.000 Zellen pro 15 ml Plasmagel mit oder ohne weitere Zellen, zugefügt sein.

Bei einer Verwendung des erfindungsgemäßen Plasmagels als Substrat für Zellkulturzwecke gemäß Anspruch 8 kann während der Gelierung vorteilhaft allogenes, vorzugsweise autologes Kollagen, z.B. aus azellulärer Dermis (Alloderm ®, Epiflex ®) gewonnen, in einer Menge von 1 bis 80 Gew-%, vorzugsweise 10 - 30 Gew-%, zugesetzt werden. Nach der homogenen Verteilung werden in den Ansatz 5 bis 5000 I.E. Thrombin, bevorzugt 500 I.E., eingerührt oder durch einen Mehrkomponentenmischer beigefügt bis die gewünschte gelartige Konsistenz erreicht und das Gemisch als Schicht ausgebracht ist. Alternativ kann das Kollagen nach der Herstellung des Plasmagelfilms bzw. nach dessen Ausbringen auf einer Kulturunterlage (z.B. in einer Zellkulturflasche) homogen ausgebracht werden. Dies kann beispielsweise durch Ausgießen oder Aufsprühen erfolgen.

Die Aufbereitung des Blutes zu erfindungsgemäßem Plasmagel ist gegenüber der Gewinnung des in der WO 00/32207 bzw. EP 592 242 beschriebenen Firbrinklebers erheblich vereinfacht und ermöglicht auch in Notfallsituationen den Einsatz von vollständig autologem Medium. Eine aufwändige Handhabung und Applikation von getrennt zu lagernden Komponenten entfällt.

Es ist besonders bevorzugt, Zelltransplantate mit dem erfindungsgemäßen Plasmagel als kurzzeitig immobilisierendem Träger zur Regeneration von zerstörtem oder geschädigtem Gewebe einzusetzen. Gemäß einer bevorzugten Ausführungsform wird daher gemäß Anspruch 10 ein Zelltransplantat aus vorzugsweise kultivierten Zellen in dem erfindungsgemäßen Plasmagel bereitgestellt.

Zur Applikation werden die gewünschten Zellen, die zuvor ggfs. z.B. durch Zentrifugieren aus der Kultur isoliert wurden, in dem Plasmagel aufgenommen. Das Mengenverhältnis von Zellen zu Plasmagel ist in weiten Grenzen variierbar und damit an verschiedenste Anforderungen anpassbar. In der Praxis richtet sich das Mengenverhältnis einerseits nach den zur Verfügung stehenden Zellen und andererseits nach der Größe der Wundoberfläche. Die Konzentration wird um so geringer sein, je weniger Zellen zur Verfügung stehen bzw. bei gegebener Zellmenge je größer die Wunde ist. Beispielsweise haben sich 30 Millionen Zellen auf 1500 cm² Wundoberfläche bewährt. Aber schon bei 2 Millionen Zellen auf 100cm² Wundoberfläche ließ sich ein gegenüber der Anwendung von Zellen ohne Plasmagel verbesserter Heileffekt beobachten. Im allgemeinen wird eine den physiologischen Verhältnissen möglichst nahekommende Konzentration angestrebt, d.h. etwa 1 bis 100 Millionen Zellen pro 15 ml Plasmagel.

Die erfindungsgemäß verwendeten Zellen können im Prinzip alle zur Regeneration von geschädigtem oder zerstörtem Gewebe geeigneten Zellen sein, u.a. embryonale oder andere Stammzellen in totipotenter oder omnipotenter Form, Melanozyten, Keratinozyten, Fibroblasten, Knorpelzellen, Adipozyten, gefäßbildende Zellen, Schleimhautzellen, Schleimhautepithelzellen (Mucosa), Urothelzellen, Muskelzellen, Nervenzellen, Zellen des Sehnen- und Bewegungsapparates, Leberzellen, Nierenzellen, Muskelzellen der glatten und quergestreiften Muskulatur, hormonproduzierende Zellen, Drüsenzellen, etc. und auch Vorläufer- oder Folgestadien in determinierter, differenzierter, vitaler, avitaler oder genetisch veränderter Form, sowie deren Derivate. Zur Behandlung von Verletzungen der Haut eignen sich z.B. alle als wundheilungsfördernd bekannten dermalen, epidermalen oder mesenchymalen Zellen. Bevorzugt werden Keratinozyten und/oder Fibroblasten oder adulte mesenchymale Stammzellen (MSC marrow stromal cells) eingesetzt.

Erfindungsgemäß werden zur Behandlung von akuten oder chronischen Wunden Zellen eingesetzt, die vorzugsweise in vitro in Zellkulturen vermehrt worden sind. Auch die Verwendung von nicht kultivierten Zellen oder Spenderzellen ist möglich, wenn keine Zeit für die Zellkultur zur Verfügung steht.

Die Gewinnung und die Kultur solcher Zellen ist dem Fachmann an sich bekannt. Ganz allgemein werden die Zellen aus einer Gewebebiopsie z.B. durch Enzymverdau freigesetzt und dann aus der Suspension isoliert. Anschließend erfolgt die Kultur in geeigneten Nährmedien bis zur gewünschten Menge. Ein für Keratinozyten besonders geeignetes Verfahren ergibt sich beispielsweise aus der WO 01/40444. Die Zellen werden abschließend in üblicher Weise geerntet. Es ist bevorzugt, die Zellen durch Zentrifugieren zu pelletieren.

Erfindungsgemäß ist es besonders bevorzugt, die einzelnen Zelltypen der Gewebebiopsie, z.B. Fibroblasten und Keratinozyten, vor der Kultur voneinander zu trennen und jede für sich zu kultivieren. Dadurch lassen sich für jede Zellart die Kulturbedingungen optimieren. Eine gleichzeitige Applikation verschiedener Zellen, beispielsweise Fibroblasten und Keratinozyten, kann in einfacher Weise durch Vermischen der aus der Zellkultur geernteten Zellen vor der Aufnahme in das Plasmagel oder durch aufeinanderfolgende Aufnahme der gewünschten Mengen der Zellen in das Plasmagel erreicht werden.

Gemäß einer weiteren besonders bevorzugten Ausführungsform ist es möglich, als Zellen ein Gewebehomogenisat aus in einem physiologisch verträglichem Medium homogenisiertem Gewebe einzusetzen. In diesem Falle kann für das Plasmagel auf den Zusatz von Calciumchloridlösung zu dem Plasma verzichtet werden, da in dem Gewebehomogenisat normalerweise bereits Calciumionen in der erforderlichen Konzentration vorhanden sind. Als physiologisch verträgliches Medium ist im Falle von Gewebehomogenisaten Ringerlactat besonders bevorzugt.

Die erfindungsgemäßen Zelltransplantate und Homogenisate können neben den kultivierten Zellen und dem Plasmagel xenogenes, autologes oder allogenes Kollagen in nativer oder säuregelöster Form, z.B. aus azellulärer Dermis (Alloderm ®, Epiflex ®) gewonnen, enthalten. Bevorzugt ist frisch aufbereitetes autologes und insbesondere allogenes Kollagen. Die Menge beträgt von 1 bis 80 Gew-%, vorzugsweise 10 - 30 Gew-%. Es ist auch vorteilhaft, wenn spezielle Nährstoffe und/oder Spurenelemente und/oder Transmittersubstanzen zugesetzt sind, die eine Hilfswirkung in der frühen Wundheilung der ersten Tage entfalten können.

Im allgemeinen erfolgt die Herstellung der Zelltransplantate oder Gewebehomogenisate vorteilhaft so, dass die Zellen bzw. das Gewebehomogenisat in einem Rührbehälter in physiologisch verträglichem Medium vorgelegt werden. Dann wird erforderlichenfalls Calciumchloridlösung zur Einstellung einer Calciumionenkonzentration im Bereich von 20 bis 60 mmol, vorzugsweise 30 bis 50 mmol zugefügt. Anschließend wird Glycerin oder Glycerinhydrat bis zu einer Endkonzentration von 1 bis 10, vorzugsweise 1 bis 6 Gew.-% eingerührt. Im Ergebnis liegen die Zellen bzw. das Homogenisat in dem erfindungsgemäß zusammengesetzten Plasmagel vor.

Nach der Applikation des erfindungsgemäßen Zelltransplantats oder Gewebehomogenisats auf die zu behandelnde Wunde wird durch die im Gewebe vorhandenen natürlichen Gerinnungsfaktoren eine weitere Verfestigung des Plasmagels unter Ausbildung einer festen Verbindung mit der Wundoberfläche ausgelöst. Das Plasmagel gewährleistet so eine sichere Adhäsion der aufzubringenden Zellen an der Wundoberfläche und benötigt keine zusätzlichen Komponenten oder exogenen Trägerstoffe. Auf die Verwendung von zusätzlichen Substanzen, z.B. Aprotinin zur Stabilisierung des Fibrinklebers, wird ausdrücklich verzichtet.

Bei der Applikation von Gewebehomogenisat hat sich die gleichzeitige oder direkt anschließende Applikation von Thrombin bewährt. Dazu werden vorzugsweise je 100 cm² Oberfläche 5 bis 5000 I.E., insbesondere 500 I.E. Thrombin in 1 ml Calciumchloridlösung mit einer Konzentration von 20 bis 60 mmol, insbesondere 30 bis 50 mmol, aufgebracht. Dies kann besonders einfach durch Applikation mit einem Mischapplikator geschehen. Soll eine Verfestigung des Plasmagels nur auf die oberste Schicht begrenzt erfolgen, ist eine direkt aufeinanderfolgende Applikation sinnvoll.

Es hat sich weiter überraschend gezeigt, dass Fibroblasten allein, ohne Keratinozyten, einen heilungsstimulierenden, d.h. die Reepithelisierung und die narbenarme Reorganisation von dermalen Gewebsanteilen unterstützenden, Effekt haben, wenn sie nach ihrer in vitro Vermehrung auf die Wunden appliziert werden. Dieser Effekt kann auch für gleichartig applizierte adulte mesenschymale Stammzellen (MSC marrow stromal cells) nachgewiesen werden.

Die vorliegende Erfindung betrifft daher weiterhin Zusammensetzungen zur Wundbehandlung, die Fibroblasten als alleinige Wundheilungsstimulatoren enthalten. Die Anwendung kann dabei sowohl in dem oben beschriebenen Plasmagel als auch direkt in dem zur Kultur verwendeten physiologischen Nährmedium erfolgen. Letztere Variante erfordert jedoch entweder, dass das Wundareal waagerecht gehalten wird oder dass eine entsprechende Wundabdeckung die aufgebrachten Fibroblasten im Wundareai festlegt. Daher ist eine Applikation in Plasmagel wegen der erleichterten Handhabung bevorzugt.

Ein großer Vorteil der erfindungsgemäßen Zelltransplantate besteht darin, dass eine Sprühapplikation nicht unbedingt erforderlich ist. Zwar ist es wegen der gleichmäßigen Verteilung und des berührungslosen und somit schmerzfreien Auftrags bevorzugt, die Zusammensetzung aufzusprühen. Dazu ist aber kein Fibrinsprühapparat erforderlich, vielmehr können handelsübliche Tropfdispenser oder Airbrushsysteme eingesetzt werden. Genauso ist auch, z.B. bei ohnehin anästhesierten Patienten, ein Aufstreichen mittels steriler Handgeräte oder ein Aufspritzen denkbar.

Die in der Intensivmedizin üblichen Dispenser können nicht nur für den Applikationsvorgang der Zellen sondern auch für die Durchmischung der pelletierten oder in Lösung befindlichen Zellen verwandt werden.

Zur Wundbehandlung wird die Wundoberfläche in an sich bekannter Weise zunächst gründlich gesäubert und steril gemacht (Wundexcision). Der Abschluss der immunologischen Phase, während derer der Körper durch entzündliche Reaktion Verunreinigungen der Wunde bekämpft, ist hierbei Grundvoraussetzung für eine schnelle und vollständige Heilung. Auf die so vorbereitete Wundoberfläche wird das erfindungsgemäße Zelltransplantat aus den kultivierten Zellen und dem Plasmagel aufgebracht. Anschließend kann die Oberfläche, soweit sinnvoll, mit an sich bekannten Abdeckungen versehen werden. Bevorzugt wird eine Abdeckung mit geeigneten interaktiven Biopolymeren.

In einer bevorzugten Ausführungsform werden auf die gereinigte Wundoberfläche Fibroblasten in Plasmagel aufgetragen und die Wunde dann gleichzeitig oder zeitversetzt mit einer zweite Schicht mit epithelialer Funktion abgedeckt. Die zweite Schicht kann beispielsweise ein Sheet mit kultivierten Keratinozyten oder Spalthaut oder gemeshte Haut sein.

Ein neuer Ansatz ist daher die Konditionierung der Wunde durch die Applikation von in der Gewebekultur vermehrten Zellen dermaler oder mesenchymaler Herkunft, um die Wundheilung in einer frühen Phase zu beschleunigen und um frühzeitig die Grundlage für einen nachfolgend zu applizierenden belastbaren, dauerhaften Hautersatz zu gewährleisten.

In einer weiteren bevorzugten Ausführungsform wird eine zunächst mit bekanntem dermalen Ersatzgewebe behandelte Wunde nach ausreichender Vaskularisierung der Neodermis erneut angefrischt und dann mit der erfindungsgemäßen Zusammensetzung behandelt. Zu den bekannten epidermalen Ersatzgeweben zählen u.a. konfluente Keratinozytensheets gemäß Rheinwald JG, Green H., Serial Cultivation of strains of human epidermal keratinocytes: the formation of keratinizing colonies from single cells, Cell 6, 331-344, 1975. Einen Überblick gibt Boyce S., Cultured Skin substitutes: a review, Tissue engineering 2(4), 255-266, 1996. Unter der Marke Integra® Artificial Skin ist eine dermale Wunddeckung von Johnson & Johnson kommerziell erhältlich.

Die erfindungsgemäßen Zelltransplantate eignen sich besonders für:
- Frische akute Wunden (z.B. Verbrennungen) von gut vaskularisiertem Status, die nach Vorkonditionierung mit temporären Deckungsmaterialien Fibroblasten als alleinige Zelltherapie erhalten und danach mit körpereigener weit expandierter Spalthaut netzartig überdeckt werden
- Frische akute Wunden (z.B. Verbrennungen) mit mehrwöchig eingeheilten Integra-Implantaten (Integra- Life Science, USA) von gutem bis mittlerem Vaskularisationstatus, die nach intraoperativer Teilresektion der Oberfläche von Integra Neodermis entstehen, danach intraoperativ mit körpereigener weit expandierter Spalthaut netzartig überdeckt und mit zusätzlichen Standard Keratinozytensheets überdeckt werden
- Frische akute Wunden (z.B. Verbrennungen) mit mehrwöchig eingeheilten Integra-Implantaten (Integra- Life Science, USA) von gutem bis mittlerem Vaskularisationstatus, auf die nach intraoperativ erfolgter Teilresektion der Oberfläche Fibroblasten appliziert werden und ein weitexpandiertes Netz aus körpereigener dünner gemeshter Spalthaut, ohne zusätzliche Applikation weiterer Zellen aus der Zellkultur, z.B. Keratinozyten, die üblicherweise zeitgleich oder zeitverzögert mitverwandt werden
- Frische akute Wunden (z.B. Verbrennungen) mit mehrwöchig eingeheilten Integra-Implantaten (Integra- Life Science, USA) von gutem bis mittlerem Vaskularisationstatus, auf die nach intraoperativ erfolgter Teilresektion der Oberfläche Fibroblasten appliziert werden
- chronische Wunden von schlecht vaskularisiertem Status (Ulcera) die in einer präepithelisierenden Heilungsphase (z.B. nach chirurgischer Resektion von altem Granulationsgewebe bei Ulcuspatienten) allein mit mesenchymalen Zellen behandelt werden, um eine Heilungsstimulierung dieser Wunden zu erreichen
- akute und/oder chronische Wunden oder schlecht heilende Wunden in der Kiefer- und HNO- Chirurgie und in der Onkologischen Chirurgie

Die Erfindung wird durch die folgenden Beispiele näher erläutert, ohne jedoch darauf beschränkt zu sein.

### Beispiel 1: Verfahren zur Isolation und Vermehrung der Fibroblasten

Biopsien von menschlichem Gewebe werden in einer steriler Petrischale von Fettgewebe befreit. Das Fettgewebe selbst kann zur Isolation von u.a. Endothelzellen, Adipozyten, Präadipozyten, Fibroblasten, Nervenzellen verwendet werden. Die Biopsien werden mit 30ml DMEM (Dulbecco modified Eagle Medium) mit Antibiotika gewaschen, in neue sterile Petrischalen überführt und die Dermis weitestgehend abpräpariert. Die präparierten Dermisstücke werden dann mit einer Schere zerkleinert ("mincing") und ein- bis mehrmals mit 30 ml Leibovitz L 15 (Gibco) mit Antibiotika gewaschen. Danach werden die Stücke in einen Erlenmeyerkolben überführt und darin durch Enzymverdau (Collagenase-A 120 - 220 U/ml und 0,1 - 0,2 %-ige Dispase in DMEM mit 1 - 15 % vorzugsweise 5 - 10 % Serum als Lösungsmittel) über Nacht bei 37 °C im CO₂-Inkubator desintegriert. Die Zellen im Überstand werden nach der Inkubation abgenommen und bei 300-500 xg, 5 - 10 Minuten abzentrifugiert. Das Pellet der Zentrifugation wird in DMEM /10 % Serum suspendiert. Die erforderliche Menge Suspensionsmedium ergibt sich aus der ermittelten Zellzahl und der Anzahl der anzulegenden Kulturen. Die Aussaat der Fibroblasten erfolgt in equilibrierte Zellkulturflaschen. Die Kulturen werden im Inkubator bei 37 °C und 5,5 - 7,5% CO₂ inkubiert.

### Beispiel 2: Verfahren zur Isolation und Vermehrung mesenchymaler Stammzellen

Humane mesenchymalen Stammzellen (hMSCs) werden aus 20-ml Knochenmarkaspiraten aus dem Beckenkamm isoliert. Ein geeignetes Vorgehen wurde von David C. Colter et al. vorgeschlagen (Rapid expansion of recycling stem cells in cultures of plastic-adherent cells from human bone marrow PNAS, März 28, 2000, Vol. 97, No. 9, 3213-3218) und von den Erfindern adaptiert. Jeweils 20 ml Aspirat wird 1:1 mit Hank's balanced salt solution (HBSS, Gibco) verdünnt und auf 10 ml eines geeigneten Gradienten (z.B. Ficoll, Percoll, Lymphoprep o.ä.) geschichtet. Nach der Zentrifugation bei 2500 xg für 30 min wird der mononukleäre Zelllayer der Grenzschicht abgenommen und in HBSS resuspendiert. Die Zellen werden nochmals bei 1500 xg über 15 min sedimentiert und dann in Komplettmedium (MEM alpha Medium w/o DN/RN Gibco, 20 % FCS) mit 100 units/ml Penicillin (GIBCO), 100 mg/ml Streptomycin (GIBCO); sowie 2 ml L-glutamine (GIBCO) aufgenommen. Die gesamte Suspension wird in Medium in eine Zellkulturflasche aufgenommen, ausgesät und bei 37 °C und 5 % CO₂ inkubiert. Nach 24 Stunden werden die nichtadhärenten Zellen durch Abgießen des Überstandes verworfen und die adhärenten Zellen 2 mal mit PBS gewaschen. Frisches Komplettmedium wird zugegeben und bei Bedarf gewechselt. Bei geeigneter Dichte werden die Zellen mit 0,25 % Trypsin /1 mM EDTA (GIBCO) geerntet und unter Verdünnung replattiert.

### Beispiel 3: Verfahren zur Isolation und Vermehrung endothelialer Zellen

Endotheliale Zellen werden aus dem Fettgewebe und der Dermis einer Hautbiopsie isoliert (EC) und nach der Methode von E. Jaffe vorgeschlagenen und von den Erfindern adaptierten Methode kultiviert (Jaffe E., Cell biology of endothelial cells. Hum Pathol 18;3 (1987) 234-239). Das Fettgewebe wird mit einer Schere zerkleinert ("mincing") und ein- bis mehrmals mit 30 ml Leibovitz L 15 (Gibco) mit Antibiotika gewaschen. Danach werden die Stücke in einen Erlenmeyerkolben überführt und darin durch Enzymverdau (Collagenase-A 120 - 220U/ml und 0,1 - 0,2 %-ige Dispase in DMEM mit 1 - 15 % vorzugsweise 5 - 10 % Serum als Lösungsmittel) über Nacht bei 37 °C im CO₂-Inkubator desintegriert. Die im Überstand befindliche Zellfraktion wird nach der Inkubation abgenommen und bei 300 - 500 xg, 5 - 10 Minuten abzentrifugiert. Das Pellet der Zentrifugation wird in DMEM / 10 % Serum suspendiert. Die dermale Pärparation wird einem analogen Enzymverdau unterworfen. Dann wird der Überstand dieser Fraktion mit dem suspendierten Pellet aus der Fettgewebepräparation vereinigt und über ein 30µm Zellsieb filtriert. Das Retentat wird in Medium suspendiert und abzentrifugiert. Im Pellet sind die durch den Enzymverdau unbeeinträchtigten Kapillarstrukturen enthalten. Diese werden in 0,05 % - 0,3 % Trypsin / 0,02 - 0,04 % EDTA und Collagenase-A 120 - 220U/ml aufgenommen und zweimal 30 Minuten bei 37 °C unter rühren inkubiert. Die im Überstand befindlichen Zellen werden nach der Inkubation abgenommen und bei 300 - 500 xg, 5- 10 Minuten abzentrifugiert. Das Pellet der Zentrifugation wird in MEM / 10 % Serum suspendiert. Die erforderliche Menge Suspensionsmedium ergibt sich aus der ermittelten Zellzahl und der Anzahl der anzulegenden Kulturen. Die Aussaat der Endothelzellen erfolgt in equilibrierte Zellkulturflaschen. Die Kulturen werden im Inkubator bei 37 °C und 5,5 - 7,5% CO₂ inkubiert. Das Kulturmedium wird erstmals am zweiten Kulturtag gewechselt. Der Mediumwechsel erfolgt nach Bedarf. Das verwendete Kulturmedium besteht aus MEM (Minimal Essential Medium, Gibco) mit 20 - 50 %, vorzugsweise 30 % Humanserum oder für das Wachstum von Endothelzellen optimiertem Serum (z.B. Hyclone), Isobutylmethylxanthine (IBMX, 33 mmol/l, Sigma), Choleratoxin (CT, 1 nmol/l, Sigma), Penicillin (50 U/ml) und Streptomycin (50 mg/ml). Zur Passage werden die Zellen in üblicher Weise mit Trypsin/EDTA resuspendiert. Für die primären Kulturen werden handelsübliche Kulturflaschen verwendet, die mit Gelatine (0,1-0,3% in phosphate-buffered saline (PBS) oder einem anderen geeigneten Coating (z.B. p(L)-Lysin, Kollagen) vorbereitet wurden.

### Beispiel 4: Verfahren zur Herstellung von autologem Plasmagel

Vollblut wird nach Standardmethoden präoperativ oder intraoperativ aus der Armvene unter Zusatz eines gebräuchlichen gerinnungshemmenden Mittels (Zitrat) entnommen und daraus Plasma nach Standardmethoden (Plasmapherese) gewonnen. Die zellulären Anteile des Blutes werden durch Zentrifugation abgetrennt, können aber je nach Fragestellung auch im Blut belassen werden. Je 15 ml Plasma werden in einen sterilen Rührbehälter überführt und auf Zimmertemperatur equilibriert. Je 10 ml Ansatz werden 1 ml physiologische Kochsalzlösung und dann unter langsamen Rühren soviel Calciumchloridlösung zugesetzt, dass die Endkonzentration von 30 - 50 mmol Calciumionen beträgt. Anschließend wird Glycerin oder Glycerinhydrat bis zu einer Endkonzentration von 1 - 10% vorzugsweise 3 - 6% zugefügt. Das Gemisch wird bis zu einer viskösen Konsistenz gerührt.

Es kann direkt auf eine durch sog. "anfrischen" vorbereitete Wunde aufgebracht werden. Das in der Wunde vorhandene Thrombin reicht aus, um das Plasmagel ausreichend zu verfestigen.

### Beispiel 5: Plasmagel mit beschleunigter Verfestigung

Wie in Beispiel 4 beschrieben wird Plasma gewonnen und je 15 ml Aliquots werden in einen sterilen Rührbehälter überführt und auf 18 bis 25 °C equilibriert. Je 10 ml Ansatz werden 1 ml physiologische Kochsalzlösung und dann unter langsamen Rühren Glycerin oder Glycerinhydrat bis zu einer Endkonzentration von 3% zugefügt. Das Gemisch wird bis zu einer viskösen Konsistenz gerührt. Zum beschleunigten Einsetzen der Gerinnung werden dem Ansatz je 10 ml Plasma 500 I.E. Thrombin in 1ml 40 mmol Calciumchloridlösung zugesetzt. Der Zusatz des Thrombins kann durch kurzes Einrühren zum Ansatz oder durch einen geeigneten Mischapplikator erfolgen. Nach dem Zusatz des Thrombins verfestigt das Gel innerhalb weniger Sekunden.

Diese Plasmagel eignet sich zur Zellkultivierung oder, wenn das Thrombin erst bei der Applikation durch einen Mischapplikator zugesetzt wird, zur Wundbehandlung vor allem an senkrechten Oberflächen.

### Beispiel 6: Zelltransplantat aus kultivierten Zellen

In das in den Beispielen 4 und 5 hergestellte Plasmagel können vitale Zellen eingebracht werden ohne dass es zu einem Auslösen der Gerinnungskaskade (Clotbildung) kommt. Die kultivierten Zellen werden hierzu durch eine zellkulturübliche geeignete Ezymbehandlung (z.B. 0,1 % Trypsin / ,05 % EDTA in PBS) geerntet und durch Zentrifugieren sedimentiert. Die Pellets werden in einer geeigneten Menge physiologischer Salzlösung resuspendiert, zu jeweils 10 ml Plasma werden 1 ml Zellen in physiologischer Salzlösung zugegeben. Dem Ansatz wird zusätzlich unter langsamen Rühren Calciumchloridlösung bis zu einer Endkonzentration von 40 mmol zugesetzt. Daran anschließend wird Glycerin oder Glycerinhydrat, je nach gewünschter Viskosität bis zu einer Endkonzentration von 1- 3% eingerührt.

Das Zelltransplanat wird mit einem jeweils geeigneten Applikator, vorzugsweise durch einen Sprühapplikator, auf die Wunde gebracht. Das in der Wunde vorhandene Thrombin reicht aus, um das Plasmagel ausreichend zu verfestigen. Da das Gel bei diesem Vorgehen recht langsam verfestigt, eignet es sich vorzugsweise für das Aufbringen von Zellen auf anteriore Körperflächen eines auf dem Rücken liegenden Patienten, da ein Sedimentieren der Zellen durch das Gel möglich ist.

### Beispiel 7: Zelltransplantat mit schnell verfestigtem Plasmagel

Die kultivierten Zellen werden durch eine zellkulturübliche geeignete Ezymbehandlung (z.B. 0,1 % Trypsin / 0,5 % EDTA in PBS) geerntet und durch Zentrifugieren sedimentiert. Die Pellets werden in einer geeigneten Menge physiologischer Salzlösung resuspendiert, und zu jeweils 10 ml Plasma 1 ml Zellen in physiologischer Salzlösung zugegeben. Dem Ansatz wird zusätzlich unter langsamen Rühren Calciumchloridlösung bis zu einer Endkonzentration von 40 mmol zugesetzt. Daran anschließend wird Glycerin oder Glycerinhydrat, je nach gewünschter Viskosität bis zu einer Endkonzentration von 1 - 3% eingerührt. Zu je 10ml Plasma werden 500 I.E. Thrombin in 1 ml 40 mmol Calciumchloridlösung zugesetzt. Der Zusatz des Thrombins kann durch kurzes Einrühren zum Ansatz oder durch einen geeigneten Mischapplikator erfolgen. Nach dem Zusatz des Thrombins verfestigt das Gel innerhalb weniger Sekunden. Da das Gel bei diesem Vorgehen schnell verfestigt, eignet es sich vorzugsweise für das Aufbringen von Zellen an Körperflächen wo ein Fixieren der Zellen im Gel nötig ist.

### Beispiel 8: Gewebehomogenisat aus frischaufbereiteter autologer humaner Spalthaut

Mit einem Dermatom (Aeskulap, Zimmer, Brennen o.ä.) wird ein Stück Spalthaut des Patienten entnommen. Die zu entnehmende Spalthautfläche richtet sich nach der zu behandelnden Fläche und der am Dermatom eingestellten Entnahmedicke. Liegt die Entnahmedicke zwischen 150 µm und 300 µm, kann von einer möglichen Flächenexpansion um das 15 bis 30-fache ausgegangen werden. Die entnommene Spalthaut wird zunächst in einer großen Menge physiologischer Salzlösung (z.B. Ringer-Lactat) mit Antibiotika gewaschen. Danach werden je cm² Haut 3ml physiologische Salzlösung, vorzugsweise NaCI-Lösung zugesetzt und der Ansatz in einen Tissue blender überführt (z.B. Medimachine, Becton Dickinson, Ultra-Turrax, IKA-Werk). Das Gewebe wird homogenisiert. Das Homogenisat wird in 10ml/cm² Haut physiologischer Pufferlösung (z.B. Ringer-Lactat) aufgenommen, gut gemischt und zentrifugiert. Der Vorgang wird zwei Mal wiederholt. Das Sediment wird je cm² Haut in 3ml physiologischer Salzlösung resuspendiert und unter Rühren mit 6ml gerinnungsfähigem Vollblut oder Blutplasma versetzt und Glycerin oder Glycerinhydrat bis zu einer Endkonzentration von 3 % zugefügt. Die gelartige Masse wird dann in einen geeigneten Applikator überführt und auf die Wunde appliziert. Gleichzeitig oder sofort nach dem Aufsprühen des Homogenisates werden auf die gesamte Fläche je 100cm² Fläche 500 I.E. Thrombin in 1 ml 40 mmol Calciumchloridlösung aufgesprüht. Anschließend lässt man das Material auf der Wunde für 5- 10 Minuten reagieren und deckt es dann mit einer geeigneten, nicht mit der Wunde verklebenden Material wie z.B. Silikonfolie ab.

Diese Ausführungsform ist besonders zur schnellen Versorgung von Wunden geeignet, da das erfindungsgemäße Homogenisat durch den Fortfall der Zellkultivierung in sehr kurzer Zeit zur Verfügung steht.

### Beispiel 9: Gewebehomogenisat aus allogener humaner Haut oder Dermis

Handelsübliche auf Abwesenheit von infektiösen Agenzien getestete Fremdhaut oder sterile azelluläre Dermis (z.B. Alloderm ®, Epiflex ®, Tutogen ®) wird gewogen und die erforderliche Menge zunächst in physiologischer Pufferlösung (z.B. Ringer-Lactat) mit Antibiotika gewaschen. Die erforderliche Gewichtsmenge richtet sich nach der notwendig zu deckenden Körperoberfläche bzw. nach der Anzahl der Kulturansätze. Die verwendete Menge des humanen Gewebes wird analog zu dem in Beispiel 8 beschriebenen Vorgehen in einer geeigneten Menge physiologischer Salzlösung aufgenommen und im Tissue blender homogenisiert. Das Homogenisat wird dann wie in Beispiel 8 beschrieben in Plasmagel eingerührt und in der beschriebenen Weise appliziert. Gleichzeitig oder sofort nach dem Aufsprühen des Homogenisates werden auf die gesamte Fläche je 100cm² 500 I.E. Thrombin in 1 ml 40 mmol Calciumchloridlösung aufgesprüht. Anschließend lässt man das Material auf der Wunde für 5 - 10 Minuten reagieren und deckt es dann mit einer geeigneten, nicht mit der Wunde verklebenden Material wie z.B. Silikonfolie ab.

Bei dieser Ausführungsform kann auf eine Gewebeentnahme beim Patienten verzichtet werden, was insbesondere bei sehr großflächigen Hautschädigungen vorteilhaft ist.

### Beispiel 10: Herstellung allogener Plasmagele

Anstelle des in den Beispielen 4 bis 9 verwendeten autologen Bluts können Blutoder Plasmakonserven von Fremdspendern eingesetzt werden. Es werden stets nach den allgemein gültigen Vorgaben für Blut und Blutprodukte hergestellte allogene Blutkonserven, Hautderivate und Zellen verwendet. Zur Herstellung des Plasmagels bzw. der Zelltransplantate wird in gleicher Weise wie in den Beispielen 4 bis 9 beschrieben, verfahren.

Plasmagele oder Gelfilme aus nicht getesteten Spenderquellen eigenen sich vorzugsweise zu in vitro Anwendungen.

### Beispiel 11: Herstellung eines kollagenhaltigen Plasmagelfilms

Dem gemäß Beispiel 4, 5 oder 10 hergestellten Plasmagel wird nach der Zugabe des Glyzerins je nach Fragestellung xenogenes, allogenes oder vorzugsweise autologes humanes, natives nicht säuregelöstes Kollagen in einer Menge von 1 - 80 Gew-% unter Rühren zugesetzt. Nach Erreichen der gewünschten Durchmischung wird in diesen Ansatz 500 I.E. Thrombin - Reaktionslösung eingerührt und das Gemisch als Schicht ausgebracht. Nach kurzer Reaktionszeit entsteht die gewünschte gelartige Konsistenz. Dieser Gelfilm wird in Abhängigkeit von der Herkunft und der Spezifikation des Kollagens auf Wunden appliziert, mit Zellen vermischt oder als Substrat für die Zellkultivierung verwandt.

## Patentansprüche

1. Plasmagel aus allogenem oder autologem Blut oder Blutderivaten, **dadurch gekennzeichnet, dass** es aus 10 Teilen Blutplasma, 0,5 bis 20 Teilen eines physiologisch verträglichen Mediums, 0,5 bis 5 Teilen Calciumchloridlösung und 1 bis 10 Teilen Glycerin oder Glycerinhydrat besteht, mit der Maßgabe, dass die Konzentration an Calciumionen im Bereich von 20 bis 60 mmol/l liegt und der Anteil Glycerin im Bereich von 1 bis 10 % beträgt.

2. Plasmagel gemäß Anspruch 1, **dadurch gekennzeichnet, dass** die Konzentration an Calciumionen im Bereich von 30 bis 50 mmol/l, insbesondere bei 40 mmol/l, liegt.

3. Plasmagel gemäß Anspruch 1 oder 2, **dadurch gekennzeichnet, dass** das physiologisch verträgliche Medium DMEM, Leibovitz L-15, Ringerlactat oder insbesondere physiologische Kochsalzlösung ist.

4. Plasmagel gemäß mindestens einem der Ansprüche 1 bis 3, **dadurch gekennzeichnet, dass** der Anteil an Glycerin 1 bis 6 % beträgt.

5. Herstellung eines Plasmagels gemäß einem der Ansprüche 1 bis 4, **dadurch gekennzeichnet, dass**
a) Blut oder Blutplasma bereitgestellt wird
b) gegebenenfalls die zellulären Anteile entfernt werden
c) je 10 Teilen Plasma unter Rühren 0,5 bis 20 Teile eines physiologisch verträglichen Mediums zugesetzt werden,
d) unter Rühren 0,5 bis 5 Teile Calciumchloridlösung zugefügt werden mit der Maßgabe, dass die Endkonzentration an Calciumionen im Bereich von 20 bis 60 mmol/l liegt
e) unter Rühren 1 bis 10 Teile Glycerin oder Glycerinhydrat zugefügt werden, wobei der Anteil Glycerin im Bereich von 1 bis 10 % eingestellt wird und das Gemisch bis zu einer mäßig viskösen Konsistenz gerührt wird.

6. Herstellung gemäß Anspruch 5, **dadurch gekennzeichnet, dass** autologes Blut verwendet wird.

7. Herstellung gemäß Anspruch 5 oder 6, **dadurch gekennzeichnet, dass** 5 bis 5000 I.E., vorzugsweise 50 bis 500 I.E. Thrombin zugefügt werden.

8. Verwendung eines Plasmagels gemäß einem der Ansprüche 1 bis 4 als Medium für die Zellkultur.

9. Verwendung gemäß Anspruch 8, **dadurch gekennzeichnet, dass** 5 bis 5000 I.E., vorzugsweise 50 bis 500 I.E. Thrombin zugefügt werden.

10. Zelltransplantat aus Zellkulturen zum Wiederaufbau von geschädigtem oder zerstörtem Gewebe, **dadurch gekennzeichnet, dass** die Zellkultur in einem Plasmagel vorliegt, das aus 10 Teilen Blutplasma, 0,5 bis 20 Teilen eines physiologisch verträglichen Mediums, 0,5 bis 5 Teilen Calciumchloridlösung und 1 bis 10 Teilen Glycerin oder Glycerinhydrat besteht, mit der Maßgabe, dass die Konzentration an Calciumionen im Bereich von 20 bis 60 mmol/l liegt und der Anteil Glycerin im Bereich von 1 bis 10 % beträgt.

11. Zelltransplantat gemäß Anspruch 10, **dadurch gekennzeichnet, dass** die Zellkultur aus in vitro kultivierten Zellen besteht.

12. Zelltransplantat gemäß Anspruch 10 oder 11, **dadurch gekennzeichnet** das Thrombin in einer Konzentration von 5 bis 5000 I.E. enthalten ist.

13. Zelltransplantat gemäß einem der Ansprüche 10 bis 12, **dadurch gekennzeichnet** das humane, insbesondere autologe Thrombozyten in einer Menge von 10.000 bis 1.000.000 Zellen enthalten sind.

14. Zelltransplantat gemäß einem der Ansprüche 10 bis 13, **dadurch gekennzeichnet** das Gewebe Haut und die Zellen dermale, epidermale oder mesenchymale Zellen sind.

15. Zelltransplantat gemäß Anspruch 14, **dadurch gekennzeichnet, dass** die Menge an Zellen der Zellkultur 1 Million bis 100 Millionen beträgt.

16. Zelltransplantat gemäß Anspruch 14 oder 15, **dadurch gekennzeichnet, dass** die Zellen Fibroblasten und/oder Keratinozyten sind.

17. Zelltransplantat gemäß einem der Ansprüche 10 bis 16, **dadurch gekennzeichnet, dass** zusätzlich frisch aufbereitetes autologes Kollagen enthalten ist.

18. Zelltransplantat gemäß einem der Ansprüche 10 bis 17, **dadurch gekennzeichnet, dass** das Plasmagel zusätzlich spezielle Nährstoffe für die Zellen, Hormone und/oder Transmittersubstanzen enthält.

19. Gewebehomogenisat zur Regeneration von geschädigtem oder zerstörtem Gewebe, **dadurch gekennzeichnet, dass** das Homogenisat sterilisiertes, in einem physiologisch verträglichem Medium homogenisiertes Gewebe in einem Plasmagel ist, das aus 10 Teilen Blutplasma, 0,5 bis 20 Teilen eines physiologisch verträglichen Mediums und 1 bis 10 Teilen Glycerin oder Glycerinhydrat besteht, wobei der Anteil Glycerin im Bereich von 1 bis 10 % beträgt.

20. Gewebehomogenisat gemäß Anspruch 19, **dadurch gekennzeichnet, dass** das physiologisch verträgliche Medium DMEM, Leibovitz L-15, physiologische Kochsalzlösung oder insbesondere Ringerlactat ist.

21. Zusammensetzung zur Behandlung von chronischen oder akuten Wunden der Haut, **dadurch gekennzeichnet, dass** in vitro kultivierte Fibroblasten in einem physiologisch verträglichen Medium als alleinige wundheilungsstimulierende Substanz enthalten sind.

22. Zusammensetzung gemäß Anspruch 21, **dadurch gekennzeichnet, dass** das physiologisch verträgliche Medium ein Plasmagel ist, das 10 Teilen Blutplasma, 0,5 bis 20 Teilen eines physiologisch verträglichen Mediums, 0,5 bis 5 Teilen Calciumchloridlösung und 1 bis 10 Teilen Glycerin oder Glycerinhydrat besteht, mit der Maßgabe, dass die Konzentration an Calciumionen im Bereich von 20 bis 60 mmol/l liegt und der Anteil Glycerin im Bereich von 1 bis 10 % beträgt.

23. Zusammensetzung gemäß Anspruch 21 oder 22, **dadurch gekennzeichnet, dass** das physiologisch verträgliche Medium eine Nährlösung ist, die der zur Kultivierung der Fibroblasten verwendeten Lösung entspricht.
